# EUROPEAN PATENT APPLICATION

(11) **EP 4 197 558 A1**
(43) Date of publication of application: **21.06.2023**
(21) Application number: 21857609.8
(22) Date of filing: 16.08.2021
(51) Int. Cl.: A61K 47/42, A61P 27/02, C07K 7/08

(54) **COMPOSITION OF DRUG AND WILD-TYPE CELL-PENETRATING PEPTIDE DERIVATIVE**

(30) Priority: 17.08.2020 CN 202010825480
(71) Applicant: Alephoson Biopharmaceuticals Ltd, NT Hong Kong (HK); Fudan University, Shanghai 201203 (CN)
(72) Inventor: WEI, Gang, Shanghai 201203 (CN); JIANG, Kuan, Shanghai 201203 (CN); LEE, Benjamin Tak Kwong, Hong Kong Science Park West Ave, HKSTP, Shatin, NT Hong Kong (CN); WANG, Yanfeng, Hong Kong Science Park West Ave, HKSTP, Shatin, NT Hong Kong (CN)
(74) Representative: Patentanwälte Olbricht Buchhold Keulertz
(86) International application number: PCT/CN2021/112687
(87) International publication number: WO 2022/037514

(57) **Abstract**

The present invention belongs to the field of pharmaceutical formulations, and relates to a composition comprising a derivative of a wild-type cell-penetrating peptide penetratin and a drug having positive charge under physiological pH conditions. The derivative of the wild-type cell-penetrating peptide penetratin is a lipophilic derivative designed by using the wild-type cell-penetrating peptide penetratin, and the molecule is positively charged as a whole under physiological pH conditions. Said composition of the present invention, as an ocular drug delivery system, can enable a drug positively charged under physiological pH conditions to be absorbed to the posterior segment of the eye by means of eye dropping, thereby providing a new route for achieving ocular delivery of a drug positively charged under physiological pH conditions.

## Description

### FIELD OF THE INVENTION

The present invention belongs to the field of pharmaceutical formulations relating to a composition for treating ocular diseases, more specifically relating to a composition comprising a derivative of a wild-type cell-penetrating peptide penetratin and a drug having positive charge under physiological pH conditions.

### BACKGROUND OF THE INVENTION

Cell-penetrating peptides (CPPs) are short peptides that are positively charged under physiological pH conditions, which could mediate covalently or non-covalently linked molecules or drug delivery systems (such as liposomes, nanoparticles, micelles, etc.) into cells (J. Controlled Release, 2019, 309: 106-124).

Since the derivatives of the wild-type cell-penetrating peptide penetratin retain the characteristic that the wild-type penetratin has a positive charge under physiological pH conditions, it is believed in the art that the derivative of the wild-type cell-penetrating peptide penetratin binds to a drug that is negatively charged under physiological pH conditions by electrostatic interaction and achieves intraocular delivery. That is, the derivative of the wild-type cell-penetrating peptide penetratin could self-assemble to form a nanocomplex through electrostatic interaction with biological macromolecular drugs such as genes, polypeptides and proteins with negative charges under physiological pH conditions, or self-assemble to form a nanocomplex with biological macromolecular drugs such as genes, polypeptides, and proteins with negative charges under physiological pH conditions in the presence of polymers, thereby achieving the intraocular delivery of the above-mentioned biological macromolecular drugs. For example, see Chinese patent application CN108976288 (A).

However, for drugs having positive charge under physiological pH conditions, it is generally believed that the drugs could not be administrated by intraocular delivery through in the manner above, as both of the drugs and the derivatives of the wild-type cell-penetrating peptide penetratin are positively charged. Therefore, in the art, it is necessary to solve the problem of how to promote the intraocular absorption of drugs having positive charge under physiological pH conditions and how to achieve intraocular delivery of said drugs.

### SUMMARY OF THE INVENTION

The purpose of the present invention is to provide a composition of artificially modified cell-penetrating peptides (CPPs) and drugs.

In this regard, the present invention provides a composition for treating ocular diseases, comprising:
(i) a drug having positive charge under physiological pH conditions, and
(ii) a derivative of a wild-type cell-penetrating peptide penetratin, the derivative of the wild-type cell-penetrating peptide penetratin has the following amino acid sequence:
   RX₁IKIWFX₂X₃RRMKWKK
wherein X₁, X₂, and X₃ represent hydrophobic amino acids selected from: naturally occurring amino acids alanine (A), valine (V), leucine (L), isoleucine (I), proline (P), phenylalanine (F), tryptophan (W), methionine (M), and non-naturally occurring amino acids α-aminobutyric acid, α-aminopentanoic acid, α-aminohexanoic acid, α-aminoheptanoic acid, and their combinations.

The composition for treating ocular diseases according to the present invention is preferably a solution or a suspension.

The composition for treating ocular diseases according to the present invention, preferably, X₁, X₂, and X₃ represent hydrophobic amino acids, and at least two of X₁, X₂, and X₃ are selected from: naturally occurring amino acids alanine (A), valine (V), leucine (L), isoleucine (I), proline (P), phenylalanine (F), tryptophan (W), methionine (M), and non-naturally occurring amino acids α-aminobutyric acid, α-aminopentanoic acid, α-aminohexanoic acid, α-aminoheptanoic acid, and their combinations.

The composition for treating ocular diseases according to the present invention, preferably, X₁, X₂ or X₃ is tryptophan (W).

The composition for treating ocular diseases according to the present invention, preferably, at least two of X₁, X₂ or X₃ are tryptophan (W).

The composition for treating ocular diseases according to the present invention, preferably, the derivative of the wild-type cell-penetrating peptide penetratin has an amino acid sequence of:
RWIKIWFQNRRMKWKK
RQIKIWFWNRRMKWKK
RQIKIWFQWRRMKWKK
RWIKIWFWNRRMKWKK
RWIKIWFQWRRMKWKK
RQIKIWFWWRRMKWKK
RWIKIWFWWRRMKWKK.

The composition for treating ocular diseases according to the present invention, preferably, the derivative of the wild-type cell-penetrating peptide penetratin has an amino acid sequence of:
RWIKIWFWNRRMKWKKK
RWIKIWFQWRRMKWKKK
RQIKIWFWWRRMKWKKK
RWIKIWFWWRRMKWKK.

The composition for treating ocular diseases according to the present invention, preferably, the drug is a peptide or protein drug.

The composition for treating ocular diseases according to the present invention, preferably, the drug is one or more selected from: nanoantibody, Ranibizumab, Aflibercept, Ocriplasmin, Bevacizumab, Adalimumab, Atezolizumab, Belimumab, Cetuximab, Dalotuzumab, Denosumab, Elotuzumab, Infliximab, Ipilimumab, Ixekizumab, Natalizumab, NISTmab, Nivolumab, Obinutuzumab, Ofatumumab, Palivizumab, Pembrolizumab, Pertuzumab, Ramucirumab, Rituximab, Trastuzumab, and Endostatin.

The composition for treating ocular diseases according to the present invention, preferably, the drug is one or more selected from: nanoantibody, Ranibizumab, Aflibercept, Ocriplasmin, Bevacizumab, and Cetuximab.

The composition for treating ocular diseases according to the present invention, preferably, the drug is one or more selected from: Ranibizumab, Aflibercept, and Bevacizumab.

The composition for treating ocular diseases according to the present invention, preferably, the nanoantibody is one or more selected from: Caplacizumab, Ozoralizumab, and Vobarilizumab.

The composition for treating ocular diseases according to the present invention, preferably, the derivative of the wild-type cell-penetrating peptide penetratin and the drug have a molar ratio of 0.1:1-50:1.

The composition for treating ocular diseases according to the present invention, more preferably, the derivative of the wild-type cell-penetrating peptide penetratin and the drug have a molar ratio of 0.5:1-35:1.

The composition for treating ocular diseases according to the present invention, even more preferably, the derivative of the wild-type cell-penetrating peptide penetratin and the drug have a molar ratio of 1:1-20:1.

The composition for treating ocular diseases according to the present invention, most preferably, the derivative of the wild-type cell-penetrating peptide penetratin and the drug have a molar ratio of 3:1-15:1.

The present invention further provides a method for treating ocular diseases in a subject in need thereof, the method comprising administering a therapeutically effective amount of the composition for treating ocular diseases according to the present invention.

The method according to the present invention, wherein the method preferably comprises administering the therapeutically effective amount of the composition according to the present invention to a patient by means of eye dropping.

The present invention further provides the use of the composition in preparing a medicament for treating ocular diseases.

### BRIEF DESCRIPTION OF THE DRAWINGS

Figure 1: Mass spectra and liquid chromatograms of the synthetic polypeptides.
Figure 2: Graphs of flow cytometry (left) and mean fluorescence intensity comparison (right) of the promoting effect of 28WP for the uptake of nanoantibody in human corneal epithelial cells.
Figure 3: Uptake of compositions of 89WP/Nanoantibody at different ratios by human retinal pigment epithelial cells.
Figure 4: Promotion of the uptake of Ocriplasmin in human retinal pigment epithelial cells by 29WP at different molar ratios.
Figure 5: Promotion of the uptake of Cetuximab in human corneal epithelial cells by 289WP at different molar ratios.
Figure 6: Quantitative evaluation of cell uptake of polypeptide/Ranibizumab compositions at different molar ratios.
Figure 7: Quantitative evaluation of cell uptake of polypeptide/Ranibizumab compositions at different molar ratios.
Figure 8: Quantitative evaluation of cell uptake of polypeptide/Ranibizumab compositions with different polypeptides.
Figure 9: Synchronous uptake behavior of the polypeptide/Ranibizumab composition in human corneal epithelial cells.
Figure 10: Synchronous uptake behavior of the polypeptide/Ranibizumab composition in human retinal pigment epithelial cells.
Figure 11: Cell uptake mechanism of eye drops of the polypeptide/Ranibizumab composition.
Figure 12: Concentrations of Ranibizumab in each tissue (blood plasma, aqueous humor, vitreous humor and retina) in rabbit at 1 h after eye drop administration of polypeptide/Ranibizumab compositions.
Figure 13: Changes of the concentration distribution of Ranibizumab in rabbit over time after eye drop administration of polypeptide/Ranibizumab compositions.
Figure 14: Concentration distribution of Aflibercept in rabbit after eye drop administration of the polypeptide/Aflibercept composition.

### DETAILED DESCRIPTION OF THE INVENTION

Ordinary eye drops have a short residence time in the conjunctival sac and have a poor absorption effect. In particular, biological macromolecular drugs such as genes, polypeptide, and proteins that are administered through local eye drops, the bioavailability in the eye is extremely low, and almost could not reach the posterior segment of the eye. Although intraocular injections and intraocular implants have high bioavailability, patients have poor compliance and are likely to cause serious complications.

In order to solve the above defects in the prior art, the present invention uses the artificially modified cell-penetrating peptide as an ocular absorption enhancer, which could be administrated as eye drops through a non-traumatic manner, and could deliver drugs having positive charge under physiological pH conditions to the eye through non-covalent binding. The cell-penetrating peptide is a short peptide having positive charge under physiological pH conditions, and the drug is positively charged under physiological pH conditions. The applicant has unexpectedly found that a combination of the cell-penetrating peptide that is positively charged under physiological pH conditions and the drug that is positively charged under physiological pH conditions could significantly promote the uptake at the cells. The composition could significantly increase the uptake of cells as compared to only using the drug that is positively charged under physiological pH conditions.

The present invention provides a composition for treating ocular diseases that comprises: (i) a drug having positive charge under physiological pH conditions, and (ii) a derivative of a wild-type cell-penetrating peptide penetratin. The derivative of the wild-type cell-penetrating peptide penetratin is a series of polypeptide derivatives with good penetrability to ocular tissues and high biological safety, which are designed and prepared by amino acid mutation methods based on naturally occurring cell-penetrating peptide penetratin. The derivative of the wild-type cell-penetrating peptide penetratin could be used to deliver drugs having positive charge under physiological pH conditions to the eye through a non-traumatic manner by mixing the drugs with said polypeptide derivative. The composition of the present invention could mediate drugs having positive charge under physiological pH conditions to efficiently permeate the absorption barrier of the eye, and promote drugs to enter the eye and reach the posterior segment of the eye, thereby improving the ocular bioavailability of drugs having positive charge under physiological pH conditions.

The wild-type cell-penetrating peptide penetratin has an amino acid sequence of:
RQIKIWFQNRRMKWKK

The penetrating ability of wild-type penetratin to the ocular tissue is improved as the hydrophobicity of the molecule increases. Therefore, on the premise of keeping the basic amino acid sequence of wild type penetratin unchanged, the penetratin derivatives according to the present invention is prepared by using amino acid mutation technology to introduce hydrophobic amino acids into the molecule, thereby enhancing the ocular tissue penetrating ability of the obtained penetratin derivatives.

Specifically, the present invention is based on the wild-type penetratin and maintains the sequence of its original basic amino acids arginine (R), lysine (K), and original hydrophobic amino acids isoleucine (I), phenylalanine (F), tryptophan (W) and methionine (M) unchanged, and using polypeptide solid-phase synthesis technology to replace hydrophilic amino acids glutamine (Q) and asparagine (N) in penetratin molecules with hydrophobic amino acids, thereby to obtain a series of polypeptide derivatives.

The characteristic of the pentetratin derivative is to replace the different hydrophilic amino acid (glutamine and asparagine) sites of the wild type pentetratin with different hydrophobic amino acids. The hydrophobic amino acids are selected from naturally occurring amino acids alanine (A), valine (V), leucine (L), isoleucine (I), proline (P), phenylalanine (F), tryptophan (W), methionine (M), and non-naturally occurring amino acids α-aminobutyric acid, α-aminopentanoic acid, α-aminohexanoic acid, α-aminoheptanoic acid, and their combinations.

The amino acid sequences of the polypeptide derivatives obtained by structural modification on the basis of wild-type penetratin are shown in Table 1, wherein the mutated amino acid is underlined. This table does not show examples of non-natural amino acid mutations, and only provide representative examples of combined mutations of different hydrophobic amino acids.

The penetrating ability of penetratin derivatives to ocular tissues is related to their hydrophobicity (lipophilicity). The stronger the hydrophobicity (lipophilicity), the stronger the penetrating ability of penetratin derivatives to ocular tissues. Therefore, penetratin derivatives prepared using other hydrophobic amino acids would also achieve enhanced ocular absorption-promoting effects.

Compared to small molecule absorption enhancers that are rarely used in the eye due to safety issues, the penetratin derivatives of the present invention, as polypeptide absorption enhancers, are easy to degrade and thus have better biological safety. On the other hand, polypeptide absorption enhancers are easy to be modified to apply to different application subject, and the penetratin derivatives of the present invention have stronger ocular absorption-promoting capabilities than naturally occurring wild-type penetratin.

**Table 1. Penetratin derivatives obtained after structural modification of wild type penetratin**

| Abbreviation | Amino acid sequence | Abbreviation | Amino acid sequence |
|---|---|---|---|
| 2-A | RAIKIWFQNRRMKWKK | 2-V | RVIKIWFQNRRMKWKK |
| 8-A | RQIKIWFANRRMKWKK | 8-V | RQIKIWFVNRRMKWKK |
| 9-A | RQIKIWFQARRMKWKK | 9-V | RQIKIWFQVRRMKWKK |
| 28-A | RAIKIWFANRRMKWKK | 28-V | RVIKIWFVNRRMKWKK |
| 29-A | RAIKIWFQARRMKWKK | 29-V | RVIKIWFQVRRMKWKK |
| 89-A | RQIKIWFAARRMKWKK | 89-V | RQIKIWFVVRRMKWKK |
| 289-A | RAIKIWFAARRMKWKK | 289-V | RVIKIWFVVRRMKWKK |
| 2-L | RLIKIWFQNRRMKWKK | 2-I | RIIKIWFQNRRMKWKK |
| 8-L | RQIKIWFLNRRMKWKK | 8-I | RQIKIWFINRRMKWKK |
| 9-L | RQIKIWFQLRRMKWKK | 9-I | RQIKIWFQIRRMKWKK |
| 28-L | RLIKIWFLNRRMKWKK | 28-I | RIIKIWFINRRMKWKK |
| 29-L | RLIKIWFQLRRMKWKK | 29-I | RIIKIWFQIRRMKWKK |
| 89-L | RQIKIWFLLRRMKWKK | 89-I | RQIKIWFIIRRMKWKK |
| 289-L | RLIKIWFLLRRMKWKK | 289-I | RIIKIWFIIRRMKWKK |
| 2-P | RPIKIWFQNRRMKWKK | 2-F | RFIKIWFQNRRMKWKK |
| 8-P | RQIKIWFPNRRMKWKK | 8-F | RQIKIWFFNRRMKWKK |
| 9-P | RQIKIWFQPRRMKWKK | 9-F | RQIKIWFQFRRMKWKK |
| 28-P | RPIKIWFPNRRMKWKK | 28-F | RFIKIWFFNRRMKWKK |
| 29-P | RPIKIWFQPRRMKWKK | 29-F | RFIKIWFQFRRMKWKK |
| 89-P | RQIKIWFPPRRMKWKK | 89-F | RQIKIWFFFRRMKWKK |
| 289-P | RPIKIWFPPRRMKWKK | 289-F | RFIKIWFFFRRMKWKK |
| 2-W | RWIKIWFQNRRMKWKK | 2-M | RMIKIWFQNRRMKWKK |
| 8-W | RQIKIWFWNRRMKWKK | 8-M | RQIKIWFMNRRMKWKK |
| 9-W | RQIKIWFQWRRMKWKK | 9-M | RQIKIWFQMRRMKWKK |
| 28-W | RWIKIWFWNRRMKWKK | 28-M | RMIKIWFMNRRMKWKK |
| 29-W | RWIKIWFQWRRMKWKK | 29-M | RMIKIWFQMRRMKWKK |
| 89-W | RQIKIWFWWRRMKWKK | 89-M | RQIKIWFMMRRMKWKK |
| 289-W | RWIKIWFWWRRMKWKK | 289-M | RMIKIWFMMRRMKWKK |
| 2-A,8-V | RAIKIWFVNRRMKWKK | 2-F,9-W | RFIKIWFQWRRMKWKK |
| 2-V,8-A | RAIKIWFANRRMKWKK | 2-W,9-F | RWIKIWFQFRRMKWKK |
| 2-V,9-L | RVIKIWFQLRRMKWKK | 2-W,9-M | RWIKIWFQMRRMKWKK |
| 2-L,9-V | RLIKIWFQVRRMKWKK | 2-M,9-W | RMIKIWFQWRRMKWKK |
| 2-L,9-I | RLIKIWFQIRRMKWKK | 2-M,8-W,9-Y | RMIKIWFWYRRMKWKK |
| 2-I,9-L | RIIKIWFQLRRMKWKK | 2-W,8-Y,9-P | RWIKIWFYPRRMKWKK |
| 2-I,8-P | RIIKIWFPNRRMKWKK | 2-Y,8-P,9-I | RYIKIWFPIRRMKWKK |
| 2-P,8-I | RPIKIWFINRRMKWKK | 2-P,8-I,9-L | RPIKIWFILRRMKWKK |
| 2-P,8-F | RPIKIWFFNRRMKWKK | 2-I,8-L,9-V | RIIKIWFLVRRMKWKK |
| 2-F,8-P | RFIKIWFPNRRMKWKK | 2-L,8-V,9-A | RLIKIWFVARRMKWKK |

In the art, isoelectric point (pl) refers to the pH value at which the net charge of a molecule, such as a protein or polypeptide, is zero. See, for example, Kentaro Tomii. Protein Properties. Encyclopedia of Bioinformatics and Computational Biology. 2019, 2: 28-33. (doi:10.1016/B978-0-12-809633-8.20266-5). Since the charge of the zwitterion changes due to different pH values of the solution, when the positive and negative charges of the zwitterion are equal, the pH value of the solution is the isoelectric point of the zwitterion. In the present invention, the drug having positive charge under physiological pH conditions is a drug with an isoelectric point higher than 7.45, such as a polypeptide or a protein drug.

In the art, physiological pH conditions refer to: under physiological conditions, pH in human is ranging from 7.35 to 7.45 with a mean value of 7.40. The pH of blood in humans is maintained within a narrow range of 7.4 ± 0.03. See, for example, Hopkins E, Sharma S. Physiology, Acid Base Balance. In: StatPearls [Internet]. StatPearls Publishing; Treasure Island (FL): Jun 16, 2019. PMID: 29939584; and Melvin E. Laski, Neil A. Kurtzman. Acid-base disorders in medicine. Disease-a-Month. 1996, 42(2): 59-125. Since the isoelectric point of the drug of the present invention is higher than the pH range in human under physiological state, the drug is positively charged under physiological pH conditions.

**Table 2. Drugs having positive charge under physiological pH conditions**

| Name of Drug | Isoelectric Point | Molecular weight (kilodaltons, kD) |
|---|---|---|
| Aflibercept | 8.8 | 115 |
| Adalimumab | 8.9 | 148 |
| Atezolizumab | 8.6 | 145 |
| Belimumab | 8.6 | 147 |
| Bevacizumab | 8.3 | 149 |
| Caplacizumab | 9.2 | 28 |
| Cetuximab | 8.8 | 152 |
| Dalotuzumab | 9.0 | 146 |
| Denosumab | 8.9 | 147 |
| Elotuzumab | 8.0 | 148 |
| Infliximab | 7.6 | 149 |
| Ipilimumab | 9.2 | 148 |
| Ixekizumab | 8.1 | 146 |
| Natalizumab | 7.8 | 149 |
| Nanoantibody | 9.6 | 13 |
| NISTmab | 9.2 | 150 |
| Nivolumab | 8.0 | 146 |
| Obinutuzumab | 8.6 | 146 |
| Ofatumumab | 9.0 | 149 |
| Ocriplasmin | 7.7 | 27 |
| Ozoralizumab | 8.8 | 38 |
| Palivizumab | 9.3 | 148 |
| Pembrolizumab | 7.6 | 149 |
| Pertuzumab | 9.0 | 148 |
| Ramucirumab | 9.1 | 147 |
| Ranibizumab | 8.8 | 48 |
| Rituximab | 9.4 | 145 |
| Trastuzumab | 9.1 | 145 |
| Vobarilizumab | 8.7 | 26 |

The composition of the present invention may be obtained by:
physically mixing the derivative of the wild-type cell-penetrating peptide penetratin and the polypeptide or protein drug having positive charge under physiological pH conditions.

The inventors have found that the composition of the present invention obtained by physical mixing could promote the absorption of the protein drug in the eye. The inventors compared the effect of the composition of the present invention in promoting cell uptake *in vitro* by cell uptake experiments. The results show that the composition having a molar ratio in the range of 0.1:1-50 for the penetratin derivative to the positively charged protein drug under physiological pH conditions could achieve good absorption of the protein drug in the eye. In addition, double-mutational (28WP, 29WP, 89WP) and triple-mutational (289WP) derivatives of all the penetratin derivatives show better absorption-promoting effect.

The protein drugs having positive charge under physiological pH conditions include Ranibizumab with an isoelectric point of 8.8, Bevacizumab with an isoelectric point of 8.8, and Aflibercept with an isoelectric point of 8.2. The composition has an optimum absorption-promoting effect, in particular at a molar ratio of 5:1 to 35: 1.

Nanoantibody is a type of novel and unique antigen-binding fragments derived from heavy chain antibodies naturally presenting in alpaca serum. Nanobodies are recombinant, single domain variable fragments derived from the heavy chain antibody of alpaca, which can selectively bind to a specific antigen. See, for example, Jovčevska I, Muyldermans S. The Therapeutic Potential of Nanobodies. BioDrugs. 2020, 34:11-26; Rubel Chakravarty, Shreya Goel, Weibo Cai. Nanoantibody: The "Magic Bullet" for Molecular Imaging? Theranostics 2014; 4(4): 386-398.

Common nanobodies include: Caplacizumab (common name: ALX 0681 or ALX-0081; trade name: Cablivi), with a molecular weight of 28 kDa and an isoelectric point of 9.2; Ozoralizumab (common name: ATN-103), with a molecular weight 38 kDa and an isoelectric point of 8.8; Vobarilizumab (common name: ALX-0061), with a molecular weight of 26 kDa and an isoelectric point of 8.7.

The present invention has confirmed *in vivo* in mice that nanoantibody can be delivered into the eye (including the cornea in the anterior segment of the eyes and the retina in the posterior segment of the eyes) by administering eye drops of the composition. In the case of using the nanoantibody alone, there is almost no absorption.

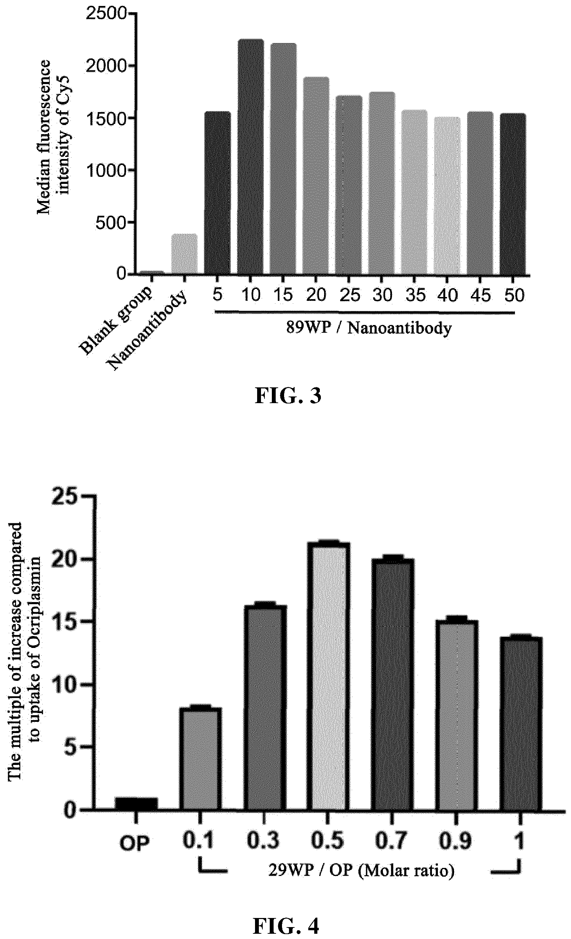

Specifically, compared with a drug that does not contain derivative 89WP of the wild-type cell-penetrating peptide penetratin and only contains the nanoantibody, the composition comprising 89WP and the nanoantibody in a molar ratio of 5:1-50:1 provides remarkable increase in cell uptake. The best uptake-promoting effect is obtained when the molar ratio of 89WP to the nanoantibody is 10:1-15:1. As shown in Figure 3, the comparison of compositions in which the ratio of 89WP to the nanoantibody (89 WP:nanoantibody) was 5:1, 10:1, 15:1, 20:1, 25:1, 30:1, 35:1, 40:1, 45:1, and 50:1, respectively. The results show that the two compositions of 89WP:nanoantibody at 10:1 and 15:1 achieved the most cell uptake.

Similarly, by administering eye drops of the composition comprising 89WP and Ranibizumab to rabbits, it was demonstrated that Ranibizumab can be delivered to the eyes (including the retina and vitreous humor in the posterior segment of the eyes) of rabbits by using the composition, which had significantly better effect than that of the control R8.

Herein, R8 is an abbreviation of poly(argnine)₈ (R means argnine), which is a classical synthetic cell-penetrating peptide. It has been reported that a homologue of R8, poly(argnine)₆ (R6), could deliver Ranibizumab and Bevacizumab into the eyes of mice by eye drop administration. See, for example: Invest Ophthalmol Vis Sci. 2017; 58(5): 2578-2590. doi: 10.1167/iovs.16-20072. It has also been reported that R8 has a better absorption-promoting effect than R6. See, for example: J Control Release. 2007;118(2): 177-84. doi: 10.1016/j.jconrel.2006.12.022.

In the present invention, the inventors use R8 as a control to prove that the effect of the present invention is better than that of R8, thereby proving that the present invention also achieves a better technical effect than that of R6 reported previously. If 89WP is not added, there is no absorption.

Specifically, the compositions of wild-type cell-penetrating peptide penetratin derivatives (R6 (poly(argnine)₆), wild-type penetratin, 2WP, 8WP, 9WP, 28WP, 29WP, 89WP, 289WP) and Ranibizumab were screened *in vitro* by using cell uptake experiments, and the molar ratio was set to 10:1. Two different kinds of cells were used, and 29WP and 289WP showed the best effect, with remarkably increased cell uptake compared with the composition which does not contain the derivative of wild-type cell-penetrating peptide penetratin and only contains Ranibizumab.

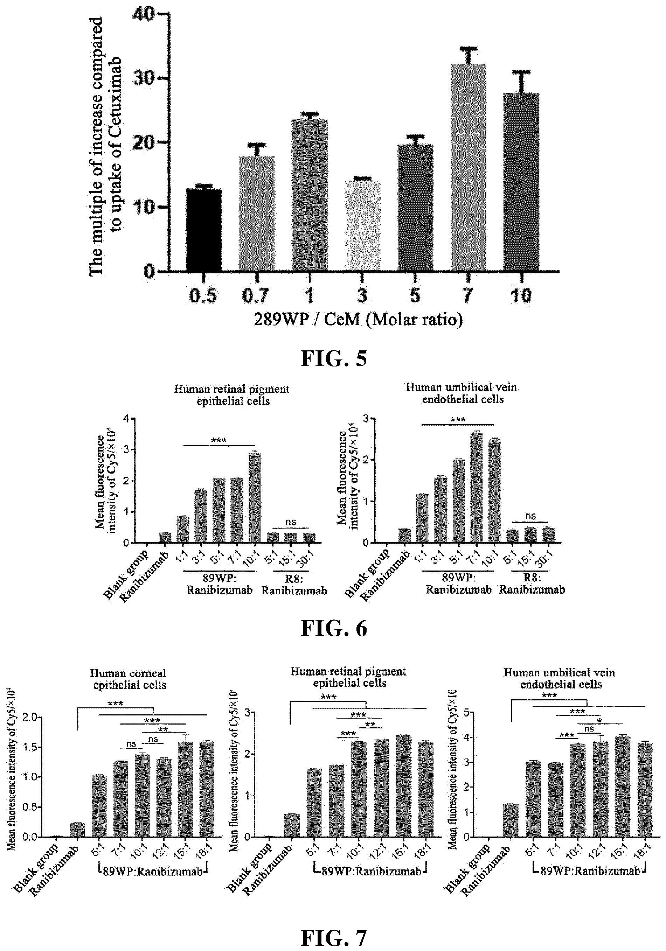

Compared with the composition containing polypeptide R6 with absorption-promoting effect and Ranibizumab (the ratio of R6:Ranibizumab is the same as the ratio of derivative of wild-type cell-penetrating peptide penetratin:Ranibizumab) reported previously (see, for example, Invest Ophthalmol Vis Sci. 2017; 58(5): 2578-2590. doi: 10.1167/iovs.16-20072, wherein poly(argnine)₆ (R6) delivers Ranibizumab and Bevacizumab into the eyes of mice by eye drop administration), as well as the composition containing wild-type penetratin and Ranibizumab (the ratio of wild-type penetratin:Ranibizumab is the same as the ratio of derivative of wild-type cell-penetrating peptide penetratin:Ranibizumab), the cell uptake of the compositions of the present invention increased significantly. As shown in Figure 6, the cell uptake increased by 13.5 and 14.7 times, respectively.

The compositions of 89WP and Ranibizumab were screened *in vitro* through cell uptake experiments, using three different cells, with the molar ratio of 89WP to Ranibizumab being 5:1-18:1. Compared with the composition that does not contain 89WP and only contains Ranibizumab, the composition of the present invention showed significant increase in cell uptake. Compared with the composition containing polypeptide R8 with absorption-promoting effect and Ranibizumab reported previously (the ratio of R8:Ranibizumab is 5:1-30:1, which is higher than the ratio of 89WP:Ranibizumab), the composition of the present invention showed significant increase in cell uptake. As shown in Figures 4 and 5, the cell uptake increased by 2.2-6.7 times.

The compositions of 289WP and Ranibizumab were screened *in vitro* through cell uptake experiments, using two different cells. It was confirmed that 289WP and Ranibizumab were synchronously taken by the cells after forming the composition (with a molar ratio of 10:1).

The compositions of 29WP and 289WP with Ranibizumab were screened *in vitro* through cell uptake experiments. The compositions were formed by using 29WP and 289WP, respectively with Ranibizumab (molar ratio 10:1). It was demonstrated in rabbits that Ranibizumab could be delivered into the eyes (including the aqueous humor in the anterior segment of the eyes, and the retina and vitreous humor in the posterior segment of the eyes) by eye drop administration. Ranibizumab was hardly absorbed if the derivative of the wild-type cell-penetrating peptide penetratin was not added. In this case, the inventors have surprisingly found that derivatives of the wild-type cell-penetrating peptide penetratin are able to interact with Ranibizumab which has positive charge under physiological pH conditions, and that derivatives of the wild-type cell-penetrating peptide penetratin are able to bring Ranibizumab into the eyes. The composition of 289WP and Aflibercept was screened *in vitro* through cell uptake experiments, wherein the composition was formed by using 289WP with Aflibercept (molar ratio 10:1). It was demonstrated in rabbits that Aflibercept could be delivered into the eyes (including the aqueous humor in the anterior segment of the eyes, and the retina and vitreous humor in the posterior segment of the eyes) by eye drop administration. Aflibercept was hardly absorbed if the derivative of the wild-type cell-penetrating peptide penetratin was not added.

Unexpected technical effects are obtained by the compositions of the present invention. Specifically, the penetratin derivatives in the compositions of the present invention retain the characteristic that the wild-type penetratin has a positive charge under physiological pH conditions, and by forming the composition non-covalently with the drug having positive charge under physiological pH conditions, unexpectedly achieve better intraocular delivery of the above drugs.

For example, by administering the composition *in vivo* in mice, the present invention demonstrates that the composition could deliver drugs such as nanoantibody to the eyes by eye drop administration, including delivering drugs such as Nanoantibody to the cornea in the anterior segment of the eyes and the retina in the posterior segment of the eyes. By administering the composition *in vivo* in rabbits, the present invention demonstrates that the composition could deliver drugs such as Ranibizumab and Aflibercept to the eyes by eye drop administration, including delivering drugs such as Ranibizumab and Aflibercept to the cornea in the anterior segment of the eyes and the retina in the posterior segment of the eyes. In the case where the drug is administered alone without using the derivative of the wild-type cell-penetrating peptide penetratin, the delivery effect cannot be obtained.

### Examples

The present invention is further illustrated below with reference to specific embodiments of the present invention without limiting the scope of protection.

### Example 1. Synthesis, purification and characterization of derivatives of wild-type cell-penetrating peptide penetratin

Polypeptide synthesis is carried out by using solid-phase synthesis technology, and the polypeptide sequences are shown in Table 3.

The crude product after lyophilization is purified by using preparative liquid chromatography, with a preparation column of Waters XBridge^{™} BEH130 Prep C18 column (19 × 250 mm, 10 µm). Subsequent purification and molecular weight characterization is carried out after lyophilizing the product.

**Table 3. Polypeptide sequence information**

| Name | Abbreviation | Sequence | Theoretical molecular weight* |
|---|---|---|---|
| Penetratin | Pe | RQIKIWFQNRRMKWKK | 2246.75 |
| Q2W-Penetratin | 2WP | RWIKIWFQNRRMKWKK | 2304.84 |
| Q8W-Penetratin | 8WP | RQIKIWFWNRRMKWKK | 2304.84 |
| N9W-Penetratin | 9WP | RQIKIWFQWRRMKWKK | 2318.86 |
| Q2W, Q8W-Penetratin | 28WP | RWIKIWFWNRRMKWKK | 2362.92 |
| Q2W, N9W-Penetratin | 29WP | RWIKIWFQWRRMKWKK | 2376.95 |
| Q8W, N9W-Penetratin | 89WP | RQIKIWFWWRRMKWKK | 2376.95 |
| Q2W, Q8W, N9W-Penetratin | 289WP | RWIKIWFWWRRMKWKK | 2435.03 |
| Poly (argnine)₆ | R6 | RRRRRR | 955.14 |

| | | | |
|---|---|---|---|
| *: The theoretical average molecular weight of the polypeptide was obtained by PeptideMass calculation tool, https://web.expasy.org/peptide mass/ | | | |

The purity of the polypeptide is investigated by using high performance liquid chromatography, and the chromatographic conditions are as follows:
Chromatographic column: YMC-Pack ODS-A column (4.6 × 150 mm, 5 µm);
Column temperature: 20 °C;
Mobile phase: Phase A is pure water (containing 0.1% TFA), and Phase B is acetonitrile (containing 0.1% TFA); the mobile phase for the derivative of the wild-type cell-penetrating peptide penetratin is 5-65% of B for 30 min, and the mobile phase for polypeptide R6 is 2-32% of B for 30 min;
Flow rate: 0.7 mL/min;
Detection wavelength: 214 nm.

The molecular weight of the polypeptide is investigated by using electrospray ionization mass spectrum (ESI-MS), and the detection conditions are as follows:
Mobile phase: methanol : pure water : formic acid = 80:19.9:0.1;
Flow rate: 0.3 mL/min;
Capillary voltage: 3000V;
Temperature and flow rate of drying gas: 350 °Cand 12 L/min.

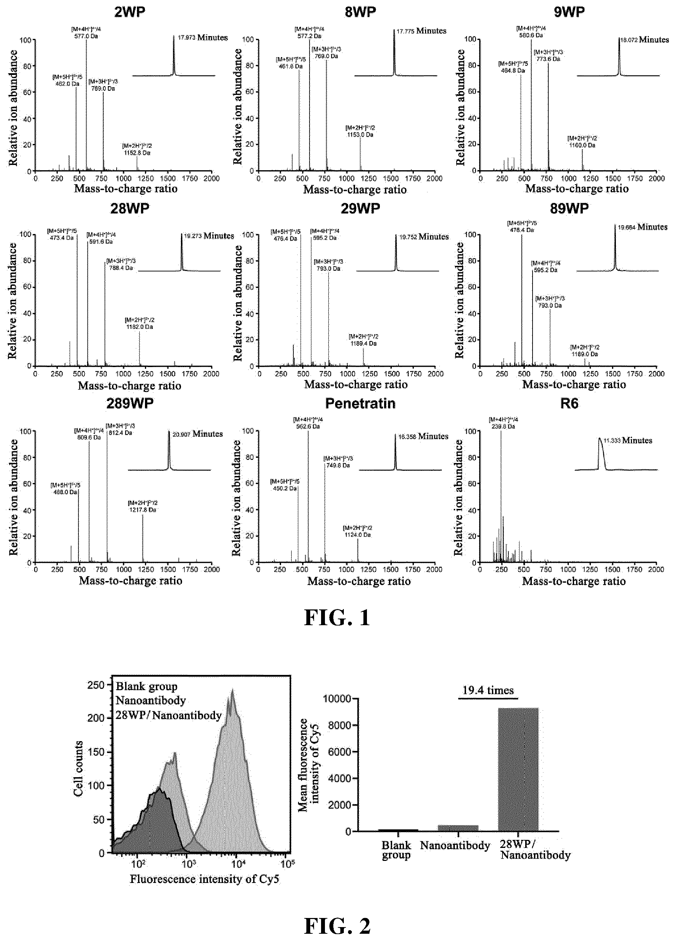

The purity and molecular weight of the polypeptides are shown in Table 4, and the detailed information of the spectra is shown in Figure 1. The liquid chromatographic purities of all the polypeptides are higher than 95% with accurate molecular weights. The polypeptide can be used for subsequent experiments.

**Table 4. Liquid chromatographic purity and molecular weight of the polypeptide**

| **Name** | **Purity (214 nm)** | **Detected molecular weight** |
|---|---|---|
| Pe | 96.47% | 2246.40 |
| 2WP | 95.94% | 2304.00 |
| 8WP | 98.11% | 2304.80 |
| 9WP | 97.84% | 2318.40 |
| 28WP | 95.71% | 2362.20 |
| 29WP | 99.22% | 2376.80 |
| 89WP | 96.16% | 2377.00 |
| 289WP | 97.65% | 2434.20 |
| R6 | 95.81% | 955.20 |

### Example 2. Polypeptides promote uptake of nanoantibody by ocular cells

Appropriate amount of nanoantibody (isoelectric point: 9.6, molecular weight: 13 kD) was dissolved in a 100 mM Na₂CO₃ solution. After the addition of fluorescent labeling reagent Sulfo-Cy5-NHS, the solution was stirred and reacted overnight at 4 °C, dialyzed in pure water at 4 °C, and lyophilized to obtain fluorescent probe Cy5-labeled Nanoantibody.

Polypeptide 28WP and Cy5-labeled nanoantibody were dissolved respectively in phosphate buffer solution (PBS, concentration: 10 mM, pH 7.4). The solution of 28WP and the solution of Cy5-labeled nanoantibody were mixed at a molar ratio of 5:1, and placed at room temperature for 24 h to obtain the composition of 28WP and nanoantibody.

Well-grown human corneal epithelial cells (HCECs) were taken and plated in a 24-well plate at 1 × 10⁴ cells/well. After 24 h of culture, the culture solution was discarded, and the composition of 28WP and nanoantibody (containing Cy5-labeled Nanoantibody at a concentration of 1 µM) was added. After incubating at 37 °C for 2 h, the drug solution was discarded, and the cells were washed 3 times with 10 mM PBS (containing 0.02 mg/mL heparin sodium), collected, and resuspended in 10 mM PBS. Flow cytometer was used for detection.

As shown in Figure 2, free nanoantibody itself was hardly taken up by human corneal epithelial cells. After the incubation of the composition formed by polypeptide 28WP and nanoantibody with cells, the cell uptake of nanoantibody was obviously improved and showed a 19.4-fold increase compared with the cell uptake of free nanoantibody.

### Example 3. Cell uptake of polypeptide/nanoantibody compositions at different ratios

89WP/nanoantibody eye drops were prepared according to the proportion in Table 5, and incubated overnight at 4 °Cfor later use.

**Table 5. 89 WP/nanoantibody eye drop composition**

| Composition (89WP:nanoantib ody, molar ratio) | 89WP (1.2 mg/mL, phosphoric acid buffer) | Cy5-nanoantibody (0.0655 mg/mL, phosphoric acid buffer) | Phosphor ic acid buffer |
|---|---|---|---|
| 5:1 | 5 µL | 100 µL | 95 µL |
| 10:1 | 10 µL | 100 µL | 90 µL |
| 15:1 | 15 µL | 100 µL | 85 µL |
| 20:1 | 20 µL | 100 µL | 80 µL |
| 25:1 | 25 µL | 100 µL | 75 µL |
| 30:1 | 30 µL | 100 µL | 70 µL |
| 35:1 | 35 µL | 100 µL | 65 µL |
| 40:1 | 40 µL | 100 µL | 60 µL |
| 45:1 | 45 µL | 100 µL | 55 µL |
| 50:1 | 50 µL | 100 µL | 50 µL |
| Cy5-nanoantibody | 0 | 100 µL | 100 µL |
| Blank | 0 | 0 | 200 µL |

The ARPE-19 cells were plated in a 24-well plate and cultured for over 24 h. The culture solution was then discarded, and 200 µL of the drug solution and 300 µL of serum-free culture solution were added into each well. After 2 h of incubation, the drug solution was discarded, and the cells were washed 3 times with PBS, digested and resuspended in PBS. Flow cytometer was used for detecting Cy5 fluorescence signals (Ex: 645 nm, Em: 665 nm).

Figure 3 shows the cell uptake under the condition that the molar ratio of 89WP to nanoantibody ranges from 5:1 to 50:1.

Figure 3 shows the uptake of 89WP/nanoantibody compositions at different ratios by ARPE-19 cells, wherein the molar ratio of 89WP to nanoantibody is 5:1, 10:1, 15:1, 20:1, 25:1, 30:1, 35:1, 40:1, 45:1 and 50:1, respectively.

The result showed that 89WP could significantly promote the cell uptake of the nanoantibody in such a ratio range, and the uptake promotion effect is better in the molar ratio range of 10:1-30:1. Thus, a preferred molar ratio of 89WP to nanoantibody is in the range of 5:1 to 35:1, more preferably 10:1 to 30: 1.

### Example 4. Polypeptides promote uptake of Ocriplasmin by ocular cells

Polypeptide 29WP and Cy5-labeled Ocriplasmin (isoelectric point: 7.7, molecular weight: 27 kD) were dissolved in citric acid buffer solution (concentration: 0.53 mg/mL), respectively. The solution of 29WP and the solution of Cy5-labeled Ocriplasmin were mixed at molar ratios of 0.1:1, 0.3:1, 0.5:1, 0.7:1, 0.9:1 and 1:1. The mixtures were placed at room temperature for 24 h to obtain compositions of 29WP and Ocriplasmin.

Well-grown human retinal pigment epithelial cells (ARPE-19) were taken and plated in a 24-well plate at 1 × 10⁴ cells/well. After 24 h of culture, the culture solution was discarded, and compositions of 29WP and Ocriplasmin (containing Cy5-labeled Ocriplasmin at a concentration of 1 µM) at different molar ratios were added. After incubating at 37 °Cfor 2 h, the drug solution was discarded, and the cells were washed 3 times with 10 mM PBS (containing 0.02 mg/mL heparin sodium), collected, and resuspended in 10 mM PBS. Flow cytometer was used for detection.

As shown in Figure 4, the composition formed by polypeptide 29WP and Ocriplasmin at a molar ratio of 0.1:1 to 1:1 could increase the uptake of Ocriplasmin in human retinal pigment epithelial cells by 7 to 21 times.

### Example 5. Polypeptides promote uptake of Cetuximab by ocular cells

Polypeptide 289WP and Cy5-labeled Cetuximab (isoelectric point: 8.8, molecular weight: 152 kD) were dissolved respectively in phosphate buffer solution (PBS, concentration: 10 mM, pH 7.4). The solution of 289WP and the solution of Cy5-labeled Cetuximab were mixed at molar ratios of 0.5:1, 0.7:1, 1:1, 3:1, 5:1, 7:1 and 10:1. The mixtures were placed at room temperature for 24 h to obtain compositions of 289WP and Cetuximab.

Well-grown human corneal epithelial cells (HCECs) were taken and plated in a 24-well plate at 1 × 10⁴ cells/well. After 24 h of culture, the culture solution was discarded, and the composition with different molar ratios of 289WP and Cetuximab (containing Cy5-labeled Cetuximab at a concentration of 1 µM) was added. After incubating at 37 °C for 2 h, the drug solution was discarded, and the cells were washed 3 times with 10 mM PBS (containing 0.02 mg/mL heparin sodium), collected, and resuspended in 10 mM PBS. Flow cytometer was used for detection.

As shown in Figure 5, the composition formed by polypeptide 289WP and Cetuximab at a molar ratio of 0.5:1 to 10:1 can increase the uptake of Cetuximab in human corneal epithelial cells by 12 to 32 times.

### Example 6. Cell uptake of polypeptide/Ranibizumab compositions at different ratios

First, Ranibizumab was fluorescently labeled. A certain amount of Ranibizumab injection was dialyzed in pure water at 4 °Cfor 3 days for desalination (with the molecular weight cut-off of 10 kD), and then lyophilized. 5 mg of lyophilized Ranibizumab was re-dissolved in a 100 mM Na₂CO₃ solution. After the addition of 0.12 mg fluorescence labeling reagent Sulfo-Cy5-NHS, the solution was stirred and reacted overnight under the condition of 4 °C. The system was then dialyzed in pure water at 4 °C for 3 days and lyophilized to obtain the fluorescently labeled Ranibizumab (Cy5-Rani).

Next, a polypeptide/Ranibizumab composition was prepared. Appropriate amount of polypeptide was weighed and dissolved by using a certain volume of commercially available Ranibizumab injection. An equal volume of commercially available Ranibizumab injection solvent was then added, and the mixture was stored at 4 °Cfor later use.

The effect of uptake of different polypeptide/Ranibizumab compositions was evaluated. Well-grown cells HCECs, ARPE-19 and HUVECs were taken and plated respectively in a 24-well plate at 1 × 10⁴ cells/well. After incubating for 24 h, the culture solution was discarded, and solutions of different compositions of polypeptide/Cy5-Rani mixture (containing 1 µM of Cy5-Rani) were added and incubated for 2 h at 37 °C. The drug solution was then discarded, and the cells were washed 3 times with 10 mM PBS (containing 0.02 mg/mL heparin sodium), collected, and resuspended in 10 mM PBS. Flow cytometer was used for detecting positive cell rate and mean fluorescence intensity, with detecting channel excitation wavelength of 638 nm and emission wavelength of 660 nm.

The results of polypeptide-mediated cell uptake of antibody at different molar ratios are as follows. After mixing the polypeptide 89WP having stronger eye penetrating ability with Cy5-labeled Ranibizumab (Cy5-Rani) at different molar ratios, the cell uptake behaviors were investigated, and the results are shown in Figures 6 and 7.

Figure 6 shows the quantitative evaluation of polypeptide-mediated cell uptake of Ranibizumab at different molar ratios. A certain amount of polypeptide is mixed with 1 µM Cy5-labeled Ranibizumab at different molar ratios (89WP/Rani, 1:1, 3:1, 5:1, 7:1, 10:1; R8/Rani, 5:1, 15:1, 30: 1) and incubated at 4 °Cfor 24 h, and then incubated with different cells for 2 h under the condition of 37 °C. Significance analysis was performed using One-Way ANOVA test, corrected by Dunnett's test (n = 3, ^{ns}*p* > 0.05, ***p < 0.001, compared to free Ranibizumab).

Figure 7 shows quantitative evaluation of polypeptide-mediated cell uptake of Ranibizumab at different molar ratios. A certain amount of polypeptide 89WP is mixed with 1 µM Cy5-labeled Ranibizumab at different molar ratios (5:1, 7:1, 10:1, 12:1, 15:1, 18:1) and incubated at 4 °Cfor 24 h, and then incubated with different cells for 2 h under the condition of 37 °C. Significance analysis was performed using One-Way ANOVA test, corrected by Tukey's test (n = 3, ^{ns}*p* > 0.05, *p < 0.05, ***p* < 0.01, ****p* < 0.001).

The results show that the polypeptide-mediated cell uptake of Ranibizumab has an ascending trend at the molar ratio of 1:1 to 10:1. According to the primary screening result, the molar ratio of 89WP to Ranibizumab is further optimized, and under the condition of 5:1 to 18:1, the polypeptide 89WP-mediated uptake of antibody in 3 kinds of cells is increased by 2.2-6.7 times compared with that of free antibody (*p* < 0.001). The cell uptake of antibody generally increased with increasing amount of polypeptide. In HCECs, there was no significant difference between the cell uptake of antibody at a molar ratio of 10:1 and those at molar ratios of 7:1 and 12:1 (*p* > 0.05). In ARPE-19 cells, the cell uptake of antibody at a molar ratio of 10:1 was significantly higher than that of the mixture at a molar ratio of 7:1 (*p* < 0.001), and lower than that of the mixture at a molar ratio of 12:1 (*p* < 0.01). In HUVECs, the cell uptake of antibody at a molar ratio of 10:1 was significantly higher than that of the mixture at a molar ratio of 7:1 (*p* < 0.001), and no significant difference from that of the mixture at a molar ratio of 12:1 (*p* > 0.05), and was slightly lower than that of the mixture at a molar ratio of 15:1 (*p* < 0.05). Generally, the polypeptide-mediated cell uptake of antibody at the molar ratio of 10:1 is significantly increased compared to the compositions with lower molar ratios, and the growth rate of the cell uptake of antibody slows down if the polypeptide amount is continuously increased. In order to improve the utilization rate of the polypeptide, the molar ratio of 10:1 is finally determined as the optimal composition formula.

### Example 7. Cell uptake of different polypeptide/Ranibizumab compositions

Compositions of different polypeptides and Ranibizumab were prepared at the molar ratio of 10:1, and their uptake behavior in HCECs and ARPE-19 cells was investigated. The results are shown in Figure 6.

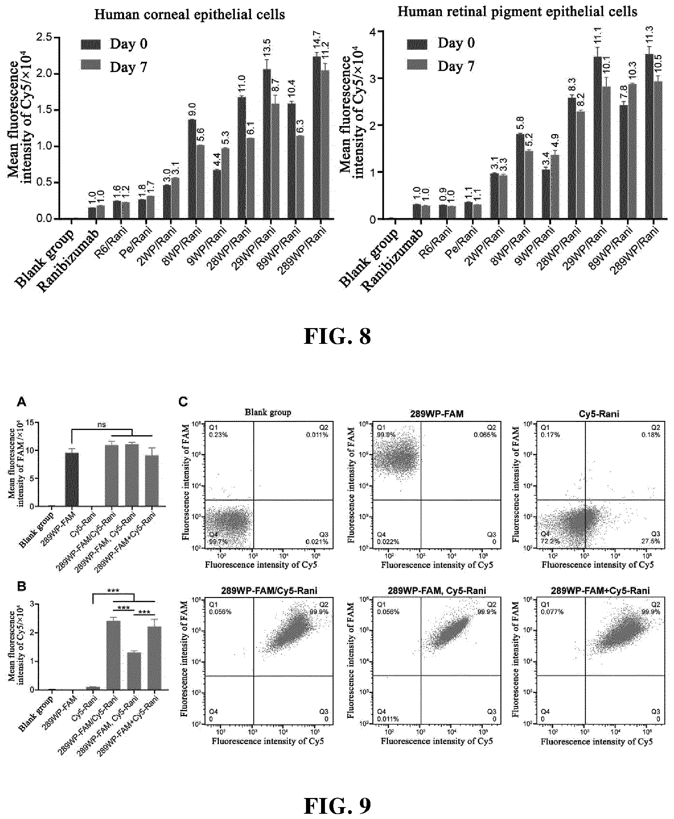

Figure 8 shows quantitative evaluation of the cell uptake of Ranibizumab mediated by different polypeptides. 10 µM of different polypeptides were mixed with 1 µM Cy5-Rani, respectively, and incubated at 4 °C for 24 h to obtain composition solutions. Before the detection, the composition solutions were incubated at 37 °C with different cells for 2 h, or the composition solutions were stored at 4 °C for 7 days and then incubated with the cells. Flow cytometer was used for detecting the mean fluorescence intensity values of cells (Ex 638 nm/Em 660 nm).

The results show that in the two cell models, all of the cell uptakes of Ranibizumab mediated by wild-type Penetratin are lower than the cell uptakes of Ranibizumab mediated by the solution of composition containing hydrophobic derived peptide. In addition, the derived peptide has enhanced lipophilicity, and then the cell uptake of rapuzumab mediated by derived peptides tend to increase. In HCECs, the polypeptides 29WP and 289WP have the strongest capacity of mediating the cell uptake of Ranibizumab, and the mean fluorescence intensities are increased by 13.5 and 14.7 times respectively, compared with free antibodies. The same results were found in ARPE-19 cells, the mean fluorescence intensities of cells in solution groups of composition based on polypeptides 29WP and 289WP are increased by 11.1 and 11.3 times, respectively. When the cell uptake behavior was investigated after storing the prepared composition solutions at 4 °Cfor 7 days, compared with the freshly prepared mixtures, the cell uptake for most solutions of composition were generally reduced, but the mean fluorescence intensities of cells based on 29WP and 289WP composition solutions were still the highest (289WP is a slightly better than 29WP) in both of the cells. Therefore, polypeptides 29WP and 289WP are selected as a preferred ocular absorption enhancer to be combined with commercially available Ranibizumab injection solution for subsequent *in vivo* pharmacokinetic evaluation.

### Example 8. Mechanism of cell uptake of polypeptide/Ranibizumab composition

The synchronous cell uptake behavior of the polypeptide and Ranibizumab was studied in this example.

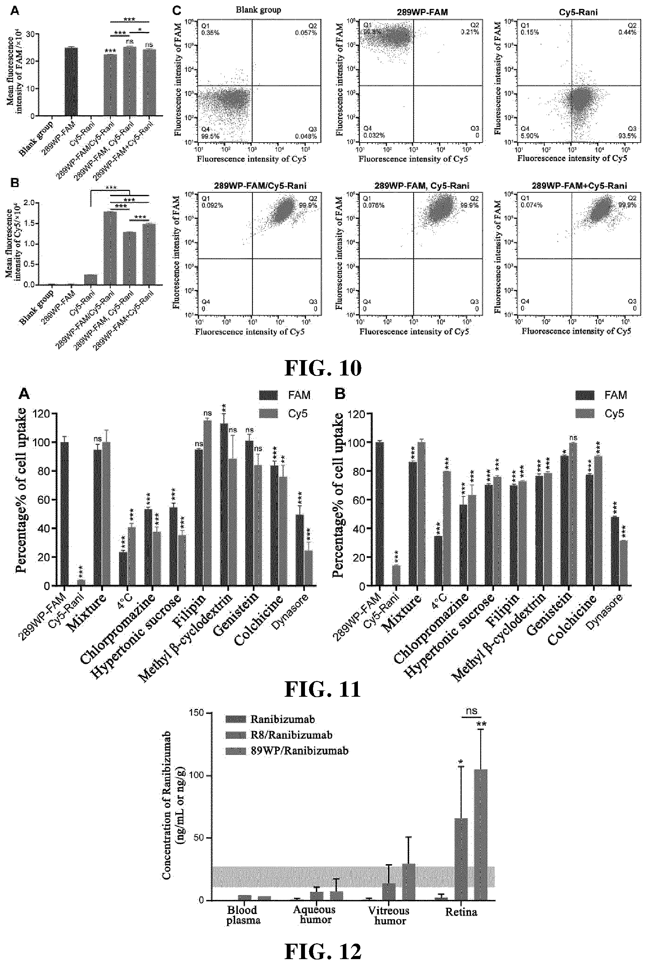

A composition of carboxyfluorescein-labeled polypeptide 289WP (289WP-FAM) and Cy5-labeled Ranibizumab (Cy5-Rani) was prepared at a molar ratio of 10:1, and the synchronous uptake behaviors of 289WP-FAM and Cy5-Rani in HCECs and ARPE-19 cells were investigated. The results are shown in Figures 9 and 10.

Figure 9 shows the synchronous uptake behavior of polypeptide/antibody in HCECs. A shows the mean fluorescence intensity of the cell uptake of carboxyfluorescein (FAM) -labeled polypeptide 289WP (289WP-FAM). B shows the mean fluorescence intensity of the cell uptake of Cy5-labeled Ranibizumab (Cy5-Rani). C shows the flow cytometry dot plots of synchronous cell uptake for 289WP-FAM and Cy5-Rani. Each composition contains 10 µM of 289WP-FAM and 1 µM of Cy5-Rani dissolving in commercially available Ranibizumab injection solvent. The detections were performed by flow cytometry (FAM, Ex 488 nm/Em 520 nm; Cy5, Ex 638 nm/Em 660 nm) after the compositions were incubated with cells at 37 °C (in 289WP-FAM/Cy5-Rani group, 289WP-FAM and Cy5-Rani were mixed, incubated at 4 °Cfor 24 h, and then incubated with cells for 1.5 h. In 289WP-FAM, Cy5-Rani group, 289WP-FAM was incubated with cells for 1.5 h, the solution was discarded, and Cy5-Rani was further incubated with the cells for 1.5 h. In 289WP-FAM + Cy5-Rani group, 289WP-FAM and Cy5-Rani were mixed and then immediately incubated with cells for 1.5 h). Significance analysis was performed using One-Way ANOVA test, corrected by Tukey's test (n = 3, ^{ns}*p* > 0.05, ****p* < 0.001).

Figure 10 shows the synchronous uptake behavior of polypeptide/antibody in ARPE-19 cells. A shows the mean fluorescence intensity of the cell uptake of the FAM-labeled polypeptide 289WP (289WP-FAM). B shows the mean fluorescence intensity of the cell uptake of Cy5-labeled Ranibizumab (Cy5-Rani). C shows the flow cytometry dot plots of synchronous cell uptake for 289WP-FAM and Cy5-Rani. Each composition contains 10 µM of 289WP-FAM and 1 µM of Cy5-Rani dissolving in commercially available Ranibizumab injection solvent. The detections were performed by flow cytometry (FAM, Ex 488 nm/Em 520 nm; Cy5, Ex 638 nm/Em 660 nm) after the compositions were incubated with cells at 37 °C(in 289 WP-FAM/Cy5-Rani group, 289WP-FAM and Cy5-Rani were mixed, incubated at 4 °C for 24 h, and then incubated with cells for 1.5 h. In 289WP-FAM, Cy5-Rani group, 289WP-FAM was incubated with cells for 1.5 h, the solution was discarded, and Cy5-Rani was further incubated with the cells for 1.5 h. In 289WP-FAM + Cy5-Rani group, 289WP-FAM and Cy5-Rani were mixed and then immediately incubated with cells for 1.5 h). Significance analysis was performed using One-Way ANOVA test, corrected by Tukey's test (n = 3, ^{ns}*p* > 0.05, *p < 0.05, ***p < 0.001).

The results show that for the cell uptake of the polypeptide (289WP-FAM), the mean fluorescence intensity value of cells for each group has no significant difference in HCECs, while in ARPE-19 cells, for cell uptake of 289WP-FAM, the mean fluorescence intensity of the composition solution group is 9/10 of that of free polypeptide. The mean fluorescence intensities of other groups have no significant difference with that of the free polypeptide, indicating that the uptakes of polypeptide 289WP-FAM have little difference in the two kinds of cells, no matter the polypeptide is administered alone or in a combination of the polypeptide and the antibody. Antibodies have little effect on the cell uptake of polypeptides. In contrast, the mean fluorescence intensities of the composition solution group (289WP-FAM/Cy5-Rani), the step-by-step uptake group (289WP-FAM, Cy5-Rani) and the freshly mixed group (289WP-FAM + Cy5-Rani) in HCECs uptake are 23.2, 12.6 and 21.3 times, respectively, compared with that of the free antibody (p < 0.001). And the mean fluorescence intensities of the mixed solution group, the step-by-step uptake group and the freshly mixed group in ARPE-19 cells are 7.4, 5.3 and 6.2 times, respectively, compared with that of the free antibody (*p* < 0.001). These show that the cell uptake effect of the antibody can be greatly improved when the polypeptide is administered synchronously with the antibody, or the cells are treated with the polypeptide firstly and then the antibody is administered (*p* < 0.001), but the synchronous administration is better than the step-by-step administration (*p* < 0.001).

### Example 9. Endocytosis mechanism of polypeptide/Ranibizumab composition

The endocytosis mechanism of the composition of polypeptide 289WP and Ranibizumab was investigated by flow cytometry at different temperatures or with the addition of endocytosis inhibitor, and the results are shown in Figure 11.

Figure 11 shows the cell uptake mechanism of polypeptide/Ranibizumab composition, wherein A and B are the effects of temperature and endocytosis inhibitor on the uptake in HCECs and ARPE-19 cells of polypeptide and Ranibizumab in the composition, respectively. The uptake amount of 289WP-FAM or Cy5-Rani in the Mixture without inhibition is used as control (100%). Significant differences were analyzed by 1-tailed ANOVA, corrected by Dunnett's test (n = 3, ^{ns}*p* > 0.05, **p* < 0.05, ***p* < 0.01, ****p* < 0.001).

The results show that, regarding the cell uptake of 289WP-FAM in the Mixture, compared with the control group (289WP-FAM, the cell uptake rate is 100%), the cell uptake of 289WP-FAM is obviously reduced in HCECs at 4 °Cor under the condition of adding an inhibitor such as chlorpromazine, hypertonic sucrose or Dynasore (inhibiting the GTPase activity of dynein), and the cell uptake rates were 23.3%, 53.2%, 54.4% and 49.4%, respectively. Similar results were also found in ARPE-19, and the corresponding cell uptake rates were 34.3%, 55.8%, 70.1% and 47.8%, respectively. The results indicate that 289WP enters cells primarily through energy-dependent, clathrin-mediated endocytosis pathways in the two kinds of cells. Regarding the cell uptake of Ranibizumab, compared with the control group (Mixture, the cell uptake rate is 100%), the cell uptake rate of Cy5-Rani is obviously reduced in HCECs at 4 °C or under the condition of adding an inhibitor such as chlorpromazine, hypertonic sucrose or Dynasore, and the cell uptake rates were 40.6%, 37.8%, 35.2% and 24.4%, respectively. Similar results were also found in ARPE-19, and the corresponding cell uptake rates were 79.6%, 63.0%, 75.7% and 31.4%, respectively.

### Example 10. Non-invasive intraocular delivery of nanoantibody mediated by polypeptide

Firstly, the nanoantibody was fluorescently labeled. 1 mg of desalted nanoantibody (isoelectric point: 9.6, molecular weight 13 kD) was dissolved in 600 uL of carbonic acid buffer solution (10 mM, pH 9.0), precooled at 4 °C, then added drop-wise with fluorescein isothiocyanate (FITC) solution (0.53 mg FITC in 53 µL DMSO), and reacted at 4 °C for 24 h. After the reaction was complete, it was dialyzed at 4 °C (MWCO 2 kD) to remove excessive FITC and inorganic salt, and lyophilized to obtain the FITC-labeled nanoantibody.

Subsequently, the distribution of the polypeptide/nanoantibody composition in the eyes of mice was investigated by the following method. 1.0 mg of FITC-labeled nanoantibody was precisely weighed and dissolved in 120 µL of physiological saline. 60 µL of the FITC-labeled nanoantibody solution was taken, uniformly mixed with 0.5 mg of cell-penetrating peptide 89WP, and placed at 4 °Cfor 24 h for use as the polypeptide/nanoantibody composition. The rest 60 µL of the FITC-labeled nanoantibody solution was used as free nanoantibody eye drops.

Mice were administered in the right eye once every 5 minutes for 3 times with 5 µL each. The concentration of FITC-labeled nanoantibody (green) contained in the free nanoantibody (Nb) solution is 8.33 µg/µL, and the concentrations of both polypeptide 89WP and FITC-labeled nanoantibody contained in the polypeptide/nanoantibody mixed solution are 8.33 µg/µL. The mice were killed at 1 h after the last administration, and performed heart perfusion by using 40 mL of physiological saline. The eyeballs were picked, washed with physiological saline, fixed in FAS eyeball stationary liquid for 24 h, and dehydrated with 15% and 30% sucrose solutions in gradient. The frozen sections of the cross section of the eye were then prepared and stained with DAPI. The distribution of fluorescence signals was observed under a laser confocal microscope.

The semi-quantitative analysis results of the nanoantibody distribution in each eye tissue after eye drop are as follows. Tables 6, 7 and 8 show the comparison of nanoantibody distributions in the cornea, corneoscleral limbus and retina of the mouse after eye drop administration, respectively.

Table 6 shows the nanoantibody distribution in the cornea after eye-dropping. The administration dose of both the free antibody eye-drop group and the polypeptide/nanoantibody solution eye-drop group are 125 µg of fluorescently labeled Nanoantibody per eye. imaged software was used to calculate the mean green fluorescence intensity value for the corneal area (FITC-labeled nanoantibody exhibits green fluorescence). The results showed that the eye drop administration of 89WP/nanoantibody composition increased the nanoantibody amount distributed in the cornea by 3.1 times, compared to that of the free nanoantibody.

**Table 6. Comparison of fluorescence signal intensity of nanoantibody distributed in cornea of mouse after eye drop administration**

| Group | Fluorescence intensity | | | Mean ± deviation | The multiple of increase in fluorescence intensity* |
|---|---|---|---|---|---|
| | 1# mouse | 2# mouse | 3# mouse | | |
| Non-administration | 2.18 | - | - | - | - |
| Free antibody eye-drop | 10.04 | 9.90 | 12.06 | 10.67±0.99 | - |
| 89WP/nanoantibody solution eye-drop | 34.37 | 34.26 | 30.98 | 33.30±1.58 | 3.11 |

| | | | | | |
|---|---|---|---|---|---|
| *: The multiple of increase in mean fluorescence intensity compared to the free antibody eye-drop group. | | | | | |

Table 7 shows the nanoantibody distribution in the corneoscleral limbus after eye-dropping. The administration dose of both the free antibody eye-drop group and the polypeptide/nanoantibody solution eye-drop group are 125 µg of fluorescently labeled nanoantibody per eye. imaged software was used to calculate the mean green fluorescence intensity value of the corneoscleral limbus region (FITC-labeled nanoantibody exhibits green fluorescence). The results showed that the eye drop administration of 89WP/nanoantibody composition increased the nanoantibody amount distributed in the corneoscleral limbus by 3.5 times, compared to that of the free nanoantibody.

**Table 7. Comparison of fluorescence signal intensity of nanoantibody distributed in corneoscleral limbus of mouse after eye drop administration**

| Group | Fluorescence intensity | | | Mean ± deviation | The multiple of increase in fluorescence intensity* |
|---|---|---|---|---|---|
| | 1# mouse | 2# mouse | 3# mouse | | |
| Non-administration | 3.96 | - | - | - | - |
| Free antibody eye-drop | 5.72 | 4.14 | 4.08 | 4.64±0.76 | - |
| 89WP/nanoantibody | 14.26 | 20.32 | 14.25 | 16.28±2.86 | 3.50 |
| solution eye-drop | | | | | |

| | | | | | |
|---|---|---|---|---|---|
| *: The multiple of increase in mean fluorescence intensity compared to the free antibody eye-drop group. | | | | | |

Table 8 shows the nanoantibody distribution in the retina after eye-dropping. The administration dose of both the free antibody eye-drop group and the polypeptide/nanoantibody solution eye-drop group are 125 µg of fluorescently labeled nanoantibody per eye. imaged software was used to calculate the mean green fluorescence intensity value for the retinal area (FITC-labeled nanoantibody exhibits green fluorescence). The results showed that compared with the free nanoantibody, the distribution of nanoantibody in the retina increased by 4.1 times when administering the 89WP/nanoantibody composition in the eye.

**Table 8. Comparison of fluorescence signal intensity of nanoantibody distributed in retina of mouse after eye drop administration**

| Group | Fluorescence intensity | | | Mean ± deviation | The multiple of increase in fluorescence intensity* |
|---|---|---|---|---|---|
| | 1# mouse | 2# mouse | 3# mouse | | |
| Non-administration | 7.92 | - | - | - | - |
| Free antibody eye-drop | 11.13 | 9.33 | 13.38 | 11.28±1.66 | - |
| 89WP/nanoantibody solution eye-drop | 40.37 | 45.84 | 51.05 | 45.75±4.36 | 4.06 |

| | | | | | |
|---|---|---|---|---|---|
| *: The multiple of increase in mean fluorescence intensity compared to the free antibody eye-drop group. | | | | | |

The above semi-quantitative analysis results of the green fluorescence signal in eye tissues show that at 1 h after the eye drop administration, the green fluorescence signal intensity of the polypeptide/nanoantibody solution eye-drop group is significantly stronger than that of the free antibody eye-drop group (p < 0.01) in all regions of the cornea, the corneoscleral limbus adjacent to retina, and retina, indicating that the addition of polypeptide 89WP into the nanoantibody solution can significantly increase the intraocular absorption of nanoantibody after eye drop administration, and particularly, the distribution of retina in the fundus is significantly increased.

### Example 11. Distribution of polypeptide/Ranibizumab composition in rabbit eyes after eye drop administration

Firstly, polypeptide/Ranibizumab (Rani) solutions were prepared by taking a certain amount of commercially available Ranibizumab injection (10 mg/mL), diluting to 5 mg/mL with a commercially available product solvent in an equal volume, dissolving R8 to 4.0 mg/mL with the resulted solution to obtain a R8/Rani mixed solution, or dissolving 89WP to 7.4 mg/mL to obtain an 89WP/Rani mixed solution. The mixed solutions were stored at 4 °C for later use. Before being used, the mixed solutions were incubated for at least 24 h at 4 °C, and kept standing at room temperature for 1 h.

The solvent formula of the commercially available Ranibizumab injection comprises the following components: 10 mM histidine hydrochloride, 10% α, α-trehalose, 0.01% Tween 20, pH 5.5.

Subsequently, the administration and tissue sampling protocol were determined.

Healthy male rabbits each about 1 kg were selected, eyes of which were ensured to be normal by ophthalmic examination before the experiment. The experimental process follows the ethical specifications of experimental animals. Each eye of the rabbit was dripped with 30 µL of drug solution containing 150 µg of Ranibizumab per eye. Tissue samples were taken at 1 h after administration.

When sampling, the rabbits were euthanized. About 1 mL of blood was immediately taken from the heart, added into a 1.5 mL anticoagulation tube, and centrifuged at 2500 g for 15 minutes at 4 °C. The supernatant was taken and stored at -20 °C for later use. Aqueous humor and vitreous humor were extracted and placed into an EP tube respectively, and stored at -20 °Cfor later use. The retinal tissue was separated, and centrifuged at 2500 g for 10 minutes at 4 °C, discarding the supernatant, washing the tissue 2 times with 10 mM PBS, and weighing. Total protein of the tissue was extracted into 10 mM PBS by a total protein extraction kit, which was then stored at -20 °Cfor later use.

The ELISA detection method was established based on the following procedure.
1) Antigen coating: hVEGF-A₁₆₅ was diluted to 1 µg/mL with a coating solution (0.05M carbonate buffer, pH 9.6). 100 µL of the diluted antigen coating solution was added to each well of an ELISA plate, and the plate was sealed and left for coating overnight at 4 °C.
2) Washing the plate: the antigen coating solution was discarded; all the coated wells were filled with a washing solution (0.02M phosphate buffer, pH 7.4, containing 0.05% Tween 20), left to standing for 30 s and dry the enzyme plate upside down, and repeated 6 times.
(3) Sealing: 250 µL of blocking solution was added to each well of the ELISA plate. The plate was sealed and incubated overnight at 4 °C.
4) The blocking solution was discarded, and the plate was washed as above.
5) Incubation of primary antibody (Ranibizumab-containing samples): 100 µL of each sample was added to wells of the ELISA plate. The plate was sealed and incubated at 37 °C for 1 h.
6) The primary antibody solution was discarded, and the plate was washed as above.
7) Incubation of secondary antibody: 100 µL of HRP-labeled secondary antibody (F (ab')2-Goat anti-Human IgG (H+L) Cross-Adsorbed Secondary Antibody, HRP) was added to each well at a dilution of 1:400. The plate was sealed and incubated at 37 °Cfor 1 h.
8) The secondary antibody solution was discarded, and the plate was washed as above.
9) Color development: 200 µL of TMB solution was added to each well. The plate was sealed and left to standing in dark for 3 minutes at 37 °C for color development. 50 µL of stop solution (2M sulfuric acid solution) was added to each well.
10) Detection: OD_{450 nm} value of each well was determined by microplate reader within 10 minutes after termination. A standard curve was drawn with the sample concentration as the horizontal coordinate and the OD_{450 nm} value as the longitudinal coordinate. The concentration of Ranibizumab contained in each sample was calculated according to the standard curve.

Ranibizumab concentrations in tissues were subsequently determined. The ELISA plate coated with antigen (hVEGF-A165) was prepared, as well as the extracted tissues (total protein extraction solution of blood plasma, aqueous humor, vitreous humor and retina). The above steps 5) to 10) were repeated, while the accompanying standard curves of sample testing were drawn to calculate the Ranibizumab concentrations in tissues.

The results of Ranibizumab concentrations in tissues detected by ELISA method are as follows. The standard curve is drawn with sample concentration corresponding to the absorbance value. Within a given concentration range (5-120 ng/mL), a good linear relation is shown between the OD_{450 nm} value detected by ELISA and the sample concentration (R² > 0.98), and within such a concentration range, the concentration of Ranibizumab can be determined by ELISA.

Distribution of Ranibizumab in ocular tissue is as follows. Ranibizumab concentration in each tissue of rabbit eye at 1 h after eye drop administration is shown in Figure 12.

Figure 12 shows Ranibizumab concentration in rabbit tissues (blood plasma, aqueous humor, vitreous humor, and retina) at 1 h after eye drop administration. The shaded region is the median effective concentration (EC₅₀) of Ranibizumab reported in the literature. Significant differences were analyzed by 1-tailed ANOVA, corrected by Tukey's test (^{ns}*p* > 0.05, *p < 0.05, **p < 0.01, compared to the Rani group).

The results show that for the free Ranibizumab eye-drop group (Rani), the drug concentrations in the aqueous humor, vitreous humor and retina of rabbit eyes can hardly be detected at 1 h after administration, which are far lower than the median effective concentration (EC₅₀) of Ranibizumab reported in the literature. For the R8/Rani solution group, after the eye drop administration, the effective therapeutic concentration is not reached in aqueous humor, and is reached at the lower limit in vitreous humor, and the effective therapeutic concentration is reached in retinal tissue, which is better than that of the free Ranibizumab group (p = 0.0377). For the group for complex of 89W/Rani, after the eye drop administration, the effective therapeutic concentration is not reached in aqueous humor, and is reached in vitreous humor and retinal tissues, which is significantly better than that of the free Ranibizumab group (p = 0.0025).

### Example 12. Pharmacokinetics of polypeptide/Ranibizumab compositions in rabbits after eye drop administration

Firstly, eye drops were prepared. Polypeptide/Ranibizumab compositions were prepared by taking a certain amount of commercially available Ranibizumab injection (10 mg/mL), diluting to 5 mg/mL with a commercially available product solvent in an equal volume, dissolving 29WP to 2.48 mg/mL with the resulted solution to obtain a composition 29WP/Rani, or dissolving 289WP to 2.54 mg/mL to obtain a composition 289WP/Rani. The compositions were stored at 4 °Cfor later use. Before being used, the compositions were incubated for at least 24 h at 4 °C, and kept standing at room temperature for 1 h.

Subsequently, the administration and tissue sampling protocol were determined. Healthy male rabbits each about 1 kg were selected, eyes of which were ensured to be normal by ophthalmic examination before the experiment. The experimental process follows the ethical specifications of experimental animals. Each eye of the rabbit was dripped with 30 µL of different composition solution containing 150 µg of Ranibizumab per eye. Ocular tissue samples were taken at 1 h, 4 h, 8 h, 12 h and 24 h after the administration.

Next, Ranibizumab concentrations in the tissues were determined. The ELISA plate coated with antigen (hVEGF-A165) was prepared, as well as the extracted tissues (total protein extraction solution of blood plasma, aqueous humor, vitreous humor and retina). Steps 5) to 10) in the ELISA detection method established in Example 11 were repeated, while the accompanying standard curves of sample testing were drawn to calculate the Ranibizumab concentrations in tissues.

The results are as follows: within a given concentration range (5-120 ng/mL), a good linear relation is shown between the OD_{450 nm} value detected by ELISA and the sample concentration of Ranibizumab (R² > 0.98), and within such a concentration range, the concentration of Ranibizumab can be determined by ELISA.

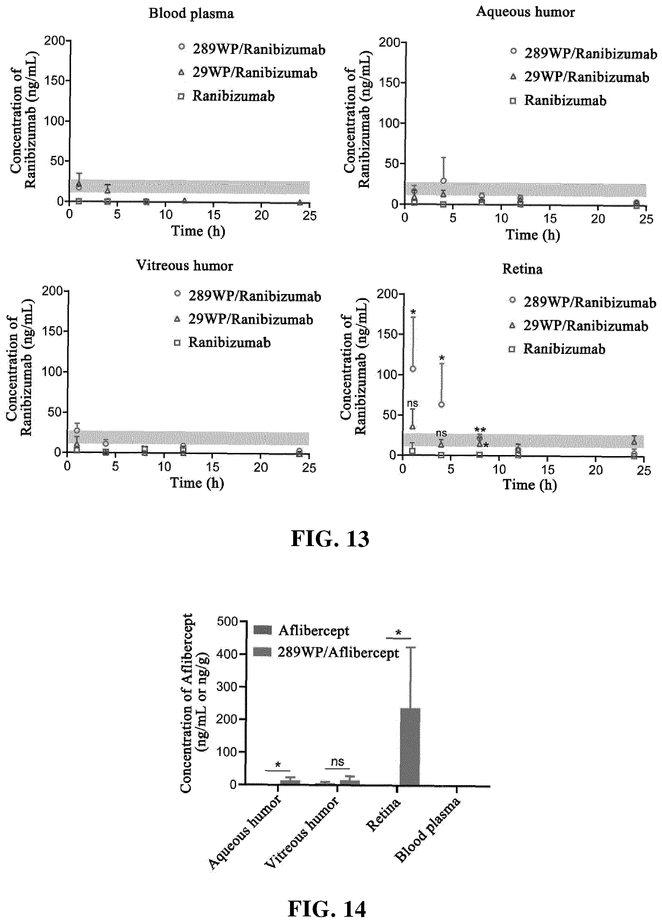

Regarding the pharmacokinetics behavior in rabbits after eye-dropping of polypeptide/Ranibizumab composition, the distribution of Ranibizumab mediated by polypeptide in each tissue in rabbits after eye drop administration was determined by ELISA, and the results are shown in Figure 13 and Table 9.

Figure 13 shows the changes of the concentration distribution of Ranibizumab in rabbit over time after eye drop administration of polypeptide/Ranibizumab compositions, with a dosage of: 76.2 µg of 289WP per eye and 150 µg of Ranibizumab per eye for 289WP/Rani composition group; 74.4 µg of 29WP per eye and 150 µg of Ranibizumab per eye for 29WP/Rani composition group; and 150 µg of Ranibizumab per eye for Rani group. Ranibizumab concentrations in tissues were determined at 1 h, 4 h, 8 h, 12 h and 24 h after the administration. The shaded region is the median effective concentration (EC₅₀) of Ranibizumab reported in the literature. Significant differences were analyzed by 1-tailed ANOVA, corrected by Tukey's test (^{ns}*p* > 0.05, **p* < 0.05, ***p* < 0.01, compared to the Rani group).

**Table 9. The concentration distribution of Ranibizumab mediated by polypeptide (ng/mL or ng/g) in rabbit after eye drop administration**

| | 289WP/Rani | | | | 29WP/Rani | | | |
|---|---|---|---|---|---|---|---|---|
| | Blood plasma | Aqueous humor | Vitreous humor | Retina | Blood plasma | Aqueou s humor | Vitreou s humor | Retina |
| 1h | 17.2±1.4 | 16.1±6.0 | 27.0±7.8 | 107.1±55. 4 | 21.7±9. 2 | 9.0±6.9 | 10.6±7. 8 | 35.8±18. 8 |
| 4h | 0.9±0.9 | 28.8±25. 2 | 11.2±4.4 | 63.4±43.6 | 14.0±5. 0 | 12.7±4. 3 | 0 | 13.7±5.2 |
| 8 h | 0 | 11.1±3.2 | 5.0±0.8 | 21.9±4.4 | 1.4±0.2 | 6.6±1.1 | 0 | 14.8±6.8 |
| 12 h | - | 8.4±3.4 | 9.1±2.3 | 7.9±5.5 | 2.0±2.0 | 6.6±1.7 | 0 | 8.8±5.1 |
| 24 h | - | 4.1±0.4 | 4.0±0.6 | 3.9±4.8 | 0.9±0.9 | 4.5±1.7 | 0 | 18.8±6.4 |

The result shows that the drug may be absorbed systemically after being dripped on the ocular surface. The systemic distribution behavior of Ranibizumab is represented by the concentration of Ranibizumab in blood plasma. At 1 h after the administration, the Ranibizumab concentrations in blood plasma are 17.2 ± 1.4 ng/mL for 289WP/Rani composition group, and 21.7 ± 9.2 ng/mL for 29WP/Rani composition group, which achieved the median effective concentration (EC₅₀) of Ranibizumab reported in the literature. However, Ranibizumab is not detected in the blood at 1 h after the eye drop administration of Rani group. At 4 h after the administration, the Ranibizumab concentrations in blood plasma for 289WP/Rani composition group and 29WP/Rani composition group are reduced to 0.9 ± 0.9 ng/mL and 14.0 ± 5.0 ng/mL, respectively. At 8 h after the administration, Ranibizumab concentration in blood plasma for each group is substantially 0. It is indicated that there is some systemic absorption of Ranibizumab mediated by polypeptide after the eye drop administration, but the Ranibizumab concentration in blood plasma is greatly reduced at 4 h after the administration, and the cleaning is basically finished at 8 h after the administration. Regarding the Ranibizumab content in the aqueous humor, Ranibizumab concentrations of 289WP/Rani composition group and 29WP/Rani composition group are within the median effective concentration (EC₅₀) range (9.0-28.8 ng/mL) of Ranibizumab as reported in the literature at 1 h and 4 h after the administration, wherein the former is slightly higher. In contrast, Ranibizumab concentration in the aqueous humor is only 1.7 ng/mL at 1 h after the administration of Rani group, indicating that it is difficult to achieve effective therapeutic concentrations of Ranibizumab in the aqueous humor when dropping alone to the eye. Regarding the concentration of Ranibizumab in vitreous humor, only 289WP/Rani composition group can reach the median effective concentration (EC₅₀) of Ranibizumab that reported in the literature (27.0 ± 7.8 ng/mL) at 1 h after the eye dropping. Ranibizumab concentration in the retina is used for characterizing the distribution of the antibody to the lesion site. For 289WP/Rani composition group, Ranibizumab concentrations in the retina are 107.1 ± 55.4 ng/g, 63.4 ± 43.6 ng/g and 21.9 ± 4.4 ng/g respectively, at 1 h, 4 h and 8 h after the administration, the mean values of which are 20.6, 12.2 and 4.2 times of that of the free antibody group, respectively (*p* < 0.05), and which are higher than or in the concentration range of the median effective concentration (EC₅₀) of Ranibizumab reported in the literature. It is indicated that the 289/Rani composition can maintain the effective concentration range of Ranibizumab in the retina within 8 h after a single eye drop administration, while the 29WP/Rani composition shows the concentration of 13.7-35.8 ng/g in the retina at 1-8 h after the eye drop administration, which can also reach the median effective concentration (EC₅₀) of Ranibizumab reported in the literature, but at a lower concentration and for a shorter duration, indicating that the intraocular delivery effects of Ranibizumab of 29WP is not as effective as 289WP. Considering the concentration of Ranibizumab in the retina > the concentration of Ranibizumab in the vitreous humor or in the aqueous humor after eye drop administration of the polypeptide/Ranibizumab composition, it is concluded that the polypeptide-mediated Ranibizumab may reach the retina through the absorption of conjunctiva → sclera → choroid → retinal.

### Example 13. Pharmacokinetics of polypeptide/Aflibercept composition in rabbits after eye drop administration

Firstly, eye drops were prepared. Polypeptide/Aflibercept composition was prepared by diluting Aflibercept (40 mg/mL) to 20 mg/mL with a commercially available Aflibercept injection solvent, dissolving polypeptide 289WP to 6.16 mg/mL with this solution, and storing at 4 °Cfor use. Before being used, the composition was incubated for at least 24 h at 4 °C, and kept standing at room temperature for 1 h.

The solvent formula of the commercial Aflibercept injection comprises the following components: 10 mM phosphate buffer, 40 mM sodium chloride, 0.03% Tween 20, 5% sucrose, pH 6.2.

Next, the administration and sampling protocol were determined. Healthy male rabbits each about 1 kg were selected, eyes of which were ensured to be normal by ophthalmic examination before the experiment. The experimental process follows the ethical specifications of experimental animals. Each eye of the rabbit was dripped with 30 µL of different composition solutions containing 600 µg of Aflibercept per eye. Ocular tissue samples were taken at 1 h after administration.

Subsequently, the Aflibercept concentrations in tissues were determined. The ELISA plate coated with antigen (hVEGF-A165) was prepared, as well as the extracted tissues (total protein extraction solution of blood plasma, aqueous humor, vitreous humor and retina). Steps 5) to 10) in the ELISA detection method established in Example 11 were repeated, while the accompanying standard curves of sample testing were drawn to calculate the Aflibercept concentrations in the tissues.

The results are as follows: within a given concentration range (0-156.25 ng/mL), a good linear relation is shown between the OD_{450 nm} value determined by ELISA and the sample concentration of Aflibercept (R² > 0.98), and within such a concentration range, the concentration of Ranibizumab can be determined by ELISA.

The polypeptide-mediated Aflibercept distribution in each tissue in rabbits after the eye drop administration was determined by ELISA, and the results are shown in Figure 14.

Figure 14 shows the concentration distribution of the polypeptide/Aflibercept solution in rabbits after the eye drop administration, with a dosage of: 600 µg of Aflibercept per eye for Afli group; 184.8 µg per eye of 289WP and 600 µg of Aflibercept per eye for 289WP/Afli composition group. The concentrations of Aflibercept in tissues were measured at 1 h after administration. Significant differences were analyzed by 2-tailed unpaired t-test (^{ns}*p* > 0.05, **p* < 0.05).

The results show that Aflibercept was not detected in blood plasma at 1 h after administration in either the free Aflibercept group or the 289WP/Afli composition group. Compared with the Afli group, Aflibercept concentrations in the aqueous humor (p < 0.05) and the vitreous humor are slightly higher after eye dropping of 289WP/Afli composition, while the concentration in the retina reaches 237.6 ± 162.2 ng/g, which is significantly higher than that of the Afli group (p < 0.05). It is indicated that polypeptide 289WP can significantly improve the ocular absorption of Aflibercept. Similar to the polypeptide/Ranibizumab eye drops, Aflibercept may be absorbed through the pathway of conjunctiva → sclera → choroid → retinal.

The above experiments demonstrate that the compositions of this invention lead to a significant increase in cell uptake compared to the case where only using the drug having positive charge under physiological pH conditions. By using the composition of the present invention, the drugs having positive charge under physiological pH conditions could be mediated to efficiently permeate the absorption barrier of the eyes, and be promoted to enter the eyes and reach the posterior segment of the eyes, thereby improving the ocular bioavailability of the drugs having positive charge under physiological pH conditions, and significantly promoting the cell uptake of the drugs having positive charge under physiological pH conditions.

## Claims

1. A composition for treating ocular diseases, comprising:
(i) a drug having positive charge under physiological pH conditions, and
(ii) a derivative of a wild-type cell-penetrating peptide penetratin, the derivative of the wild-type cell-penetrating peptide penetratin has the following amino acid sequence:
RX₁IKIWFX₂X₃RRMKWKK
wherein X₁, X₂, and X₃ represent hydrophobic amino acids selected from: naturally occurring amino acids alanine (A), valine (V), leucine (L), isoleucine (I), proline (P), phenylalanine (F), tryptophan (W), methionine (M), and non-naturally occurring amino acids α-aminobutyric acid, α-aminopentanoic acid, α-aminohexanoic acid, α-aminoheptanoic acid, and their combinations.

2. The composition for treating ocular diseases according to claim 1, wherein the composition is a solution or a suspension.

3. The composition for treating ocular diseases according to claim 1, wherein X₁, X₂, and X₃ represent hydrophobic amino acids, and at least two of X₁, X₂, and X₃ are selected from: naturally occurring amino acids alanine (A), valine (V), leucine (L), isoleucine (I), proline (P), phenylalanine (F), tryptophan (W), methionine (M), and non-naturally occurring amino acids α-aminobutyric acid, α-aminopentanoic acid, α-aminohexanoic acid, α-aminoheptanoic acid, and their combinations.

4. The composition for treating ocular diseases according to claim 1, wherein X₁, X₂ or X₃ is tryptophan (W).

5. The composition for treating ocular diseases according to claim 1, wherein at least two of X₁, X₂ or X₃ are tryptophan (W).

6. The composition for treating ocular diseases according to claim 1, wherein the derivative of the wild-type cell-penetrating peptide penetratin has an amino acid sequence of:
RWIKIWFQNRRMKWKK
RQIKIWFWNRRMKWKK
RQIKIWFQWRRMKWKK
RWIKIWFWNRRMKWKK
RWIKIWFQWRRMKWKK
RQIKIWFWWRRMKWKK
RWIKIWFWWRRMKWKK.

7. The composition for treating ocular diseases according to claim 1, wherein the derivative of the wild-type cell-penetrating peptide penetratin has an amino acid sequence of:
RWIKIWFWNRRMKWKKK
RWIKIWFQWRRMKWKKK
RQIKIWFWWRRMKWKKK
RWIKIWFWWRRMKWKK.

8. The composition for treating ocular diseases according to claim 1, wherein the drug is a peptide or protein drug.

9. The composition for treating ocular diseases according to claim 1, wherein the drug is one or more selected from: nanoantibody, Ranibizumab, Aflibercept, Ocriplasmin, Bevacizumab, Adalimumab, Atezolizumab, Belimumab, Cetuximab, Dalotuzumab, Denosumab, Elotuzumab, Infliximab, Ipilimumab, Ixekizumab, Natalizumab, NISTmab, Nivolumab, Obinutuzumab, Ofatumumab, Palivizumab, Pembrolizumab, Pertuzumab, Ramucirumab, Rituximab, Trastuzumab, and Endostatin.

10. The composition for treating ocular diseases according to claim 1, wherein the drug is one or more selected from: nanoantibody, Ranibizumab, Aflibercept, Ocriplasmin, Bevacizumab, and Cetuximab.

11. The composition for treating ocular diseases according to claim 1, wherein the drug is one or more selected from: Ranibizumab, Aflibercept, and Bevacizumab.

12. The composition for treating ocular diseases according to claim 9, wherein the nanoantibody is one or more selected from: Caplacizumab, Ozoralizumab, and Vobarilizumab.

13. The composition for treating ocular diseases according to claim 1, wherein the derivative of the wild-type cell-penetrating peptide penetratin and the drug have a molar ratio of 0.1:1-50:1.

14. The composition for treating ocular diseases according to claim 1, wherein the derivative of the wild-type cell-penetrating peptide penetratin and the drug have a molar ratio of 0.5:1-35:1.

15. The composition for treating ocular diseases according to claim 1, wherein the derivative of the wild-type cell-penetrating peptide penetratin and the drug have a molar ratio of 1:1-20:1.

16. The composition for treating ocular diseases according to claim 1, wherein the derivative of the wild-type cell-penetrating peptide penetratin and the drug have a molar ratio of 3:1-15:1.

17. A method for treating ocular diseases in a subject in need thereof, the method comprising administering a therapeutically effective amount of the composition for treating ocular diseases according to any one of claims 1-16.

18. The method according to claim 2, wherein the method comprises administering the therapeutically effective amount of the composition according to any one of claims 1-16 to a patient by means of eye dropping.

19. Use of the composition according to any one of claims 1-16 for the manufacture of a medicament for treating ocular diseases.
